(19) Europäisches Patentamt
European Patent Office
Office européen des brevets

(11) Veröffentlichungsnummer: **0 048 370**
**B1**

# (12) EUROPÄISCHE PATENTSCHRIFT

(45) Veröffentlichungstag der Patentschrift: **18.05.83**

(21) Anmeldenummer: **81107011.9**

(22) Anmeldetag: **07.09.81**

(51) Int. Cl.³: **C 07 C 61/40,** C 07 C 51/363, C 07 C 51/377, C 07 C 69/753, C 07 C 67/307, C 07 C 67/317, C 07 C 51/62, C 07 C 49/567, C 07 C 45/63, C 07 C 120/00, C 07 C 121/75

(54) **Verfahren zur Herstellung von trans-3-(Z-2-Chlor-2-aryl-vinyl)-2,2-dimethyl-cyclopropan-1-carbonsäure-derivaten, neue Zwischenprodukte hierfür, Verfahren zu deren Herstellung und Verwendung von Zwischenprodukten in Schädlingsbekämpfungsmitteln.**

(30) Priorität: **18.09.80 DE 3035149**

(43) Veröffentlichungstag der Anmeldung: **31.03.82 Patentblatt 82/13**

(45) Bekanntmachung des Hinweises auf die Patenterteilung: **18.05.83 Patentblatt 83/20**

(84) Benannte Vertragsstaaten: **AT BE CH DE FR GB IT LI NL SE**

(56) Entgegenhaltungen:
**DE-A-2 827 101**
**DE-A-2 844 271**

(73) Patentinhaber: **BAYER AG, Zentralbereich Patente, Marken und Lizenzen, D-5090 Leverkusen 1, Bayerwerk (DE)**

(72) Erfinder: **Fuchs, Rainer, Dr., Roeberstrasse 8, D-5600 Wuppertal 1 (DE)**
Erfinder: **Stendel, Wilhelm, Dr., In den Birken 55, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von trans-3-(Z-2-Chlor-2-aryl-vinyl)-2,2-dimethyl-cyclopropan-1-carbonsäurederivaten, neue Zwischenprodukte hierfür, Verfahren zu deren Herstellung und Verwendung von Zwischenprodukten in Schädlingsbekämpfungsmitteln

Die Erfindung betrifft ein neues Verfahren zur Herstellung von trans-3-(Z-2-Chlor-2-aryl)-vinyl)-2,2-dimethyl-cyclopropan-1-carbonsäure-derivaten, neue Zwischenprodukte hierfür, ein Verfahren zu deren Herstellung und die Verwendung von Zwischenprodukten in Schädlingsbekämpfungsmitteln, insbesondere in Insektiziden und Akariziden.

Es ist bereits bekannt geworden, dass Gemische der (±)-cis- und (±)-trans-Formen von 3-(E/Z-2-Chlor-2-(4-chlorphenyl)-vinyl)-2,2-dimethyl-cyclopropan-1-carbonsäure-(±)-(α-cyano-4-fluor-3-phenoxy-benzyl)-ester insektizid und akarizid wirksam sind (vgl. DE-OS 2 730 515).

Weiter sind Diastereomerengemische von Z- bzw. E-trans-3-(2-Chlor-2-(e-chlor-phenyl)-vinyl)-2,2-dimethyl-cyclopropan-1-carbonsäure-(α-cyano-4-fluor-3-phenoxy-benzyl)-ester Gegenstand der deutschen Patentanmeldung P 2 936 864.

In der vorgenannten Patentanmeldung ist die Auftrennung von E/Z-Isomerengemischen zu den E- bzw. Z-Diastereomeren von trans-3-(2-Chlor-2-(4-chlor-phenyl)-vinyl)-2,2-dimethyl-cyclopropan-1-carbonsäure durch zwei Extraktionsverfahren beschrieben. Diese Trennverfahren sind jedoch wegen des hohen apparativen Aufwandes für die Trennung grösserer Substanzmengen wenig geeignet.

Es wurde nun ein Verfahren zur Herstellung von trans-3-(Z-2-Chlor-2-(4-aryl)-vinyl)-2,2-dimethyl-cyclopropan-1-carbonsäure-derivaten der Formel I

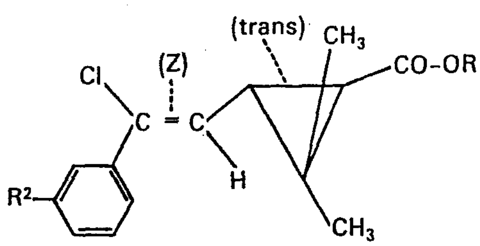

(I)

gefunden, in welcher

$R_2$ für Wasserstoff oder Chlor steht,

R für Wasserstoff, ein Alkalimetall oder Erdalkalimetalläquivalent, ein gegebenenfalls substituiertes Ammoniumion, Alkyl oder gegebenenfalls durch Alkyl, Alkenyl, Alkinyl, Halogen und/oder Cyano substituiertes Phenoxybenzyl steht,

dadurch gekennzeichnet, dass man trans-3-(1,2-Dihalogen-2-chlor-2-(4-chlor-phenyl)-ethyl)-2,2-dimethyl-cyclopropan-1-carbonsäure-derivate der Formel II

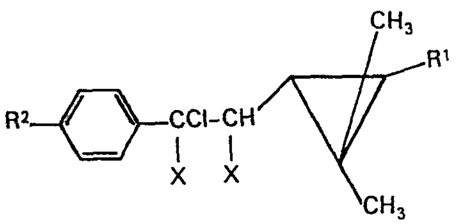

(II)

in welcher

$R^2$ für Wasserstoff oder Chlor steht,

$R^1$ für Cyano, Acetyl, Chlorcarbonyl oder für den Rest -COOR steht, worin R die oben angegebene Bedeutung hat, und

X für Chlor oder Brom steht,

mit einem zur Eliminierung von zwei vicinalen Halogenatomen geeigneten Enthalogenierungsmittel gegebenenfalls in Gegenwart eines Verdünnungsmittels bei Temperaturen zwischen 0 und 150°C umsetzt und gegebenenfalls die Produkte der Formel I a

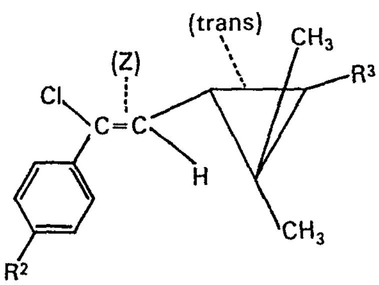

(I a)

in welcher

$R^2$ für Wasserstoff oder Chlor und

$R^3$ für Cyano, Acetyl oder Chlorcarbonyl stehen,

nach üblichen Methoden in die Verbindungen der Formel (I) umwandelt oder auch Verbindungen der Formel (I) nach üblichen Methoden in andere Verbindungen der Formel (I) umwandelt.

Die Erfindung betrifft weiter als neue Verbindungen die trans-3-(1,2,2-Trichlor-2-aryl-ethyl)-2,2-dimethyl-cyclopropan-1-carbonsäure-derivate der Formel III

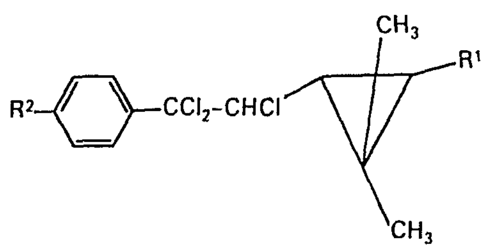

(III)

in welcher

$R^1$ und $R^2$ die unter Formel (II) angegebene Bedeutung haben.

Man erhält die neuen Verbindungen der Formel (III), wenn man trans-3-(E/Z-2-Chlor-2-aryl-vinyl)-2,2-dimethyl-cyclopropan-1-carbonsäurederivate der Formel IV

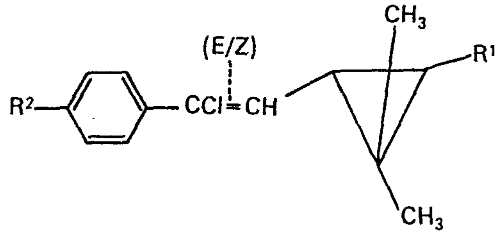

(IV)

in welcher
$R^1$ und R2 die unter Formel (II) angegebene Bedeutung hat, mit Chlor gegebenenfalls in Gegenwart eines Verdünnungsmittels bei Temperaturen zwischen -50 und +50°C umsetzt und gegebenenfalls anschliessend die Produkte der Formel III a

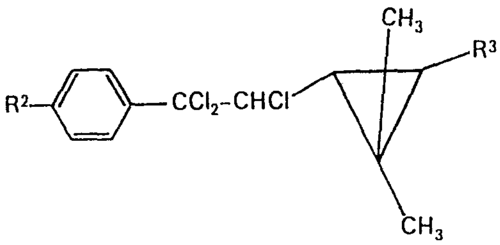

(III a)

in welcher
$R^2$ für Wasserstoff oder Chlor und
$R^3$ für Cyano, Acetyl oder Chlorcarbonyl stehen, nach üblichen Methoden in die Verbindungen der Formel (III), in welcher $R^3$ für -COOR steht, umwandelt.

Die Erfindung betrifft ferner die Verwendung von Verbindungen der Formel (III), in welcher $R^1$ für gegebenenfalls durch Alkyl, Alkenyl, Alkinyl, Halogen und/oder Cyano substituiertes Phenoxybenzyloxycarbonyl steht als Wirkstoffkomponenten in insektiziden und akariziden Mitteln, insbesondere in Ektoparasitiziden.

Überraschenderweise können nach dem erfindungsgemässen Verfahren die Verbindungen der Formel (I) mit Z-Konfiguration ausgehend von E,Z-Diastereomerengemischen der Formel (IV) mit beliebig hohem Anteil an den E-konfigurierten Isomeren in sehr guten Ausbeuten erhalten werden, wobei der Anteil an den E-Isomeren auf weniger als 5% verringert werden kann.

Der Reaktionsablauf beim erfindungsgemässen Verfahren zur Herstellung von Verbindungen der Formel (I) kann beispielsweise bei Verwendung von trans-3-(1,2,2-Trichlor-2-(4-chlor-phenyl)-ethyl)-2,2-dimethyl-cyclopropan-1-carbonsäurenitril und Zink als Ausgangsstoffen durch folgendes Formelschema skizziert werden:

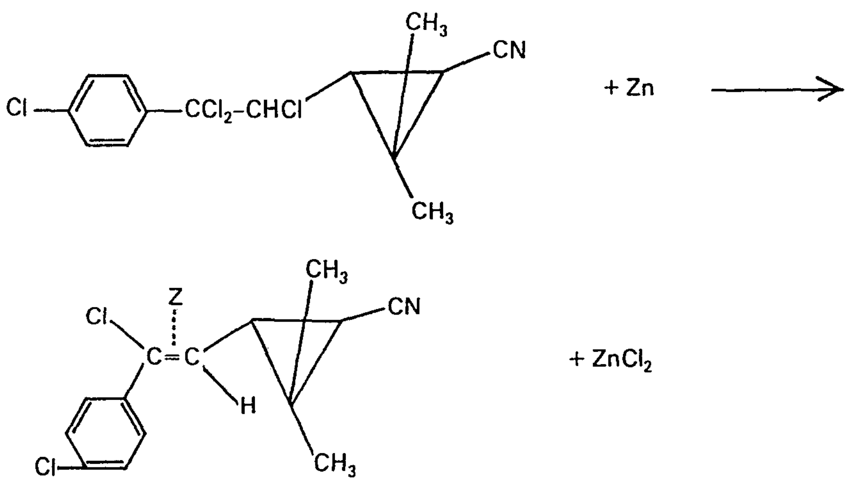


Enthalogenierungsmittel, welche beim erfindungsgemässen Verfahren eingesetzt werden können, sind in der Literatur beschrieben [vgl. Houben-Weyl, Methoden der organischen Chemie, 4. Auflage (1972) Band 5/1 b, S. 180–204, Thieme-Verlag, Stuttgart].

Als geeignete Enthalogenierungsmittel seien genannt:

(a) Metalle, wie z.B. Natrium, Kalium, Magnesium, Eisen, Nickel, Kupfer, Zink oder Legierungen hiervon;

(b) Alkalimetall-iodide oder -bromide, wie z.B. Natriumiodid und -bromid, sowie Kaliumiodid und -bromid;

(c) reduzierende Metallverbindungen, wie z.B. Chrom(II)-sulfat und Kupfer(I)bromid, sowie

(d) Phosphor(III)verbindungen, wie z.B. Triphenylphosphin und Phosphorigsäuretri-di-methylamid.

Für das erfindungsgemässe Verfahren wird vorzugsweise Zink als Enthalogenierungsmittel verwendet.

Das erfindungsgemässe Verfahren zur Herstellung von Verbindungen der Formel (I) wird vorzugsweise unter Verwendung von Verdünnungsmitteln durchgeführt. Hierzu gehören neben Wasser vor allem organische Lösungsmittel, insbesondere Kohlenwasserstoffe, wie z.B. Benzol, Toluol oder Xylol, Ether, wie z.B. Diethyl- und Dibutylether, Glycoldimethylether, Diglycoldimethylether, Tetrahydrofuran und Dioxan, Ketone wie z.B. Aceton, Methyl-ethylketon, Methyl-isopropylketon und Methyl-isobutylketon, Carbonsäuren und deren Ester, wie z.B. Essigsäure und deren Methyl- und Ethylester, Carbonsäureamide, wie z.B. Dimethylformamid und Dimethylacetamid, sowie Alkohole, wie z.B. Methanol, Ethanol, Propanole und Butanole.

Die Reaktionstemperatur wird beim Verfahren zur Herstellung von Verbindungen der Formel (I) im allgemeinen zwischen 0 und 150°C, vorzugsweise zwischen 20 und 120°C gehalten.

Das Verfahren wird bei Normaldruck oder geringfügig erhöhtem oder vermindertem Druck, im allgemeinen zwischen 0,1 und 10 bar, durchgeführt.

Zur Durchführung des erfindungsgemässen Verfahrens setzt man je Mol Ausgangsverbindung der Formel (II) 1 bis 2 Mol vorzugsweise 1 bis 1,5 Mol Enthalogenierungsmittel ein. In einer bevorzugten Ausführungsform wird das Enthalogenierungsmittel in einem der oben angegebenen Verdünnungsmittel vorgelegt und die Ausgangsverbindung der Formel (II) langsam dazu gegeben. Das Reaktionsgemisch wird dann vorzugsweise bei erhöhter Temperatur bis zum Ende der Umsetzung gerührt.

Die Aufarbeitung kann nach üblichen Methoden durchgeführt werden, beispielsweise wird das Lösungsmittel, falls es mit Wasser mischbar ist, abdestilliert und der Rückstand mit Wasser und einem mit Wasser praktisch nicht mischbaren organischen Lösungsmittel, wie z.B. Methylenchlorid geschüttelt. Die organische Phase wird abgetrennt, mit Wasser gewaschen und getrocknet. Die Produkte der Formel (I) verbleiben nach Abdestillieren des Lösungsmittels als fester oder öliger Rückstand.

Wie bereits erwähnt können dem erfindungsgemässen Verfahren vor- oder nachgeschaltet Verbindungen der Formel (I) oder (Ia) bzw. der Formel (III) oder (IIIa) nach üblichen Methoden «umfunktionialisiert», d.h. in andere Verbindungen der Formeln (I) bzw. (III) umgewandelt werden, wobei sich nur die Bedeutungen der funktionellen Gruppen $R^1$ bzw. $R^2$ ändern.

Solche Umwandlungen sind beispielsweise

(a) Verseifungen von Säurechloriden oder Cyanoverbindungen der Formeln Ia bzw. IIIa ($R^2$: -CO-Cl, -CN) zu den entsprechenden Carbonsäuren, beispielsweise durch Erhitzen mit wässrigen Alkalilaugen;

(b) Veresterungen von Säurechloriden der Formel Ia bzw. IIIa ($R^3$: -CO-Cl) durch Umsetzung mit Alkoholen bzw. durch Umsetzung mit gegebenenfalls substituierten Phenoxybenzaldehyden in Gegenwart wasserlöslicher Cyanide;

(c) Haloform-Reaktionen von Acetylverbindungen der Formeln Ia bzw. IIIa ($R^3$: $-CO-CH_3$) zu den entsprechenden Carbonsäuren, beispielsweise durch Umsetzung mit wässriger Natriumhypochloritlösung, oder auch

(d) Umwandlungen von Carbonsäurederivaten der Formel (I), wie z.B. Verseifung der Niederalkylester und Umsetzung der so erhaltenen Carbonsäuren mit Thionylchlorid zu den entsprechenden Säurechloriden nach üblichen Methoden.

Die beim erfindungsgemässen Verfahren zur Herstellung von Verbindungen der Formel (I) als Ausgangsstoffe zu verwendenden trans-3-(1,2-Dihalogen-2-chlor-2-aryl-ethyl)-2,2-dimethyl-cyclopropan-1-carbonsäure-derivate sind durch Formel (II) definiert. Vorzugsweise steht darin

$R^2$ für Wasserstoff oder Chlor

$R^1$ für Cyano, Acetyl, Chlorcarbonyl oder für den Rest -COOR, worin

R für Wasserstoff, Natrium, Kalium, $C_1$-$C_4$-Alkyl oder für gegebenenfalls durch Cyano und/oder Fluor-substituierte meta-Phenoxy-benzyl steht.

Als Ausgangsstoffe besonders bevorzugt sind die neuen Verbindungen der Formel (III), worin

$R^2$ für Chlor und

$R^1$ die oben als bevorzugt angegebene Bedeutung hat.

Als Beispiele für die Verbindungen der Formel (III) seien genannt:

trans-3-(1,2,2-Trichlor-2-(4-chlor-phenyl)-ethyl)-2,2-dimethyl-cyclopropan-1-carbonsäure, deren Chlorid, deren Natrium- und Kalium-salz, deren Nitril, deren Methyl-, Ethyl-, n- und iso-Propyl-, n-, iso-, sek.- und tert.- Butyl-ester, deren 3-Phenoxy-benzylester, deren α-Cyano-3-phenoxy-benzylester, deren 4-Fluor-3-phenoxy-benzylester und deren α-Cyano-4-fluor-3-phenoxy-benzylester sowie (trans-3-(1,2,2-Trichlor-2-(4-chlorphenyl)-ethyl)-2,2-dimethyl-1-cyclopropyl)-methyl-keton.

Man erhält die Ausgangsverbindungen der Formel (II) – diese umfasst auch die neuen Verbindungen der Formel (III) – auf an sich bekannte Weise durch Umsetzung von trans-3-(E/Z-2-Chlor-2-(4-chlor-phenyl)-vinyl)-2,2-dimethyl-cyclopropan-1-carbonsäure-derivaten der Formel IV (oben) mit Chlor bzw. Brom, vorzugswseise in Gegenwart eines inerten Verdünnungsmittels, wie z.B. Methylenchlorid, Chloroform oder Tetrachlorkohlenstoff, bei Temperaturen zwischen -50 und +50°C, vorzugsweise zwischen -20 und +30°C.

Das Halogen wird gewöhnlich in leichtem Überschuss bis zu 30%, vorzugsweise zwischen 5 und 20% eingesetzt. Das Halogenierungsprodukt der Formel (II) erhält man nach Abdestillieren des Lösungsmittels und Digerieren des Rückstandes mit Kohlenwasserstoffen, wie z.B. Hexan, in kristalliner Form.

Die als Vorprodukte zu verwendenden trans-3-(E/Z-2-Chlor-2-(4-chlor-phenyl)-vinyl)-2,-2-dimethyl-cyclopropan-1-carbonsäure-derivate sind durch Formel (IV) definiert. Vorzugsweise hat darin $R^1$ die oben als bevorzugt angegebene Bedeutung.

Als Beispiele für die Verbindungen der Formel (IV) seien genannt:

trans-3-(E/Z-2-Chlor-2-(4-chlor-phenyl)-vinyl)-2,2-dimethyl-cyclopropan-1-carbonsäure, deren Chlorid, deren Natrium- und Kalium-salz, deren Nitril, deren Methyl-, Ethyl-, n- und iso-Propyl-, n-, iso-, sek.- und tert.-Butyl-ester, deren 3-Phenoxybenzylester, deren α-Cyano-3-phenoxy-benzylester, deren 4-Fluor-3-phenoxy-benzylester und deren α-Cyano-4-fluor-3-phenoxy-benzylester sowie (trans-3-(E/Z-2-Chlor-2-(4-chlor-phenyl)-vinyl)-2,2-dimethyl-1-cyclopropyl)-methylketon.

Die Verbindungen der Formel (IV) können nach einem in der Patentliteratur beschriebenen Verfahren (vgl. europäische Patentanmeldung Nr. 79 101864.1) aus trans-3-Formyl-2,2-dimethyl-cyclopropan-1-carbonsäure-derivaten und α-Chlor-4-chlor-benzyl-phosphonsäure-dialkylestern hergestellt werden.

Die vorliegende Patentanmeldung betrifft auch die Verwendung von Zwischenprodukten der Formel (III), insbesondere von trans-3-(1,2,2-Trichlor-2-(4-chlor-phenyl)-ethyl)-2,2-dimethyl-cyclopropan-1-carbonsäure-phenoxybenzylestern der Formel IIIb

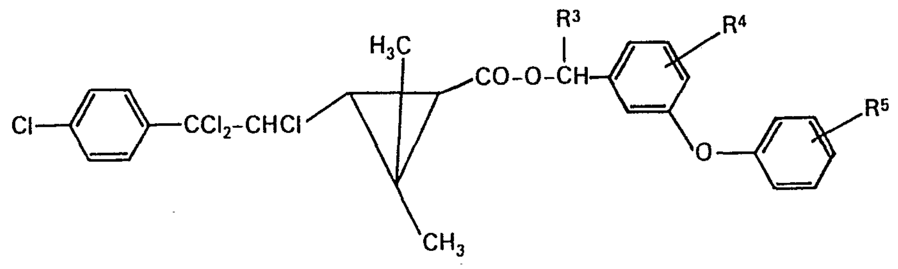

(III b)

in welcher
$R^3$ für Wasserstoff oder Cyano steht und
$R^4$ und $R^5$ für Wasserstoff und/oder Fluor stehen, in Schädlingsbekämpfungsmitteln.

Als Beispiele für die Verbindungen der Formel (IIIb) seien genannt:

trans-3-(1,2,2-Trichlor-2-(4-chlor-phenyl)-ethyl)-2,2-dimethyl-cyclopropan-1-carbonsäure-3-phenoxy-benzylester,
-α-cyano-3-phenoxy-benzylester,
-4-fluor-3-phenoxy-benzylester,
-3-(4-fluor-phenoxy)-benzylester,
-α-cyano-4-fluor-3-phenoxy-benzylester und
-α-cyano-3-(4-fluor-phenoxy)-benzylester.

Beispiel 1:
Halogenierung

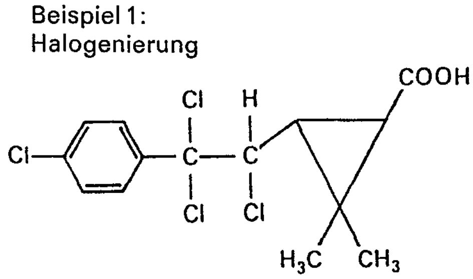

28,5 g (0,1 Mol) trans-3-(E,Z-2-Chlor-2-(4-chlor-phenyl)-vinyl)-2,2-dimethyl-cyclopropan-1-carbonsäure mit einem E:Z-Verhältnis von 60:40 werden in 150 ml Tetrachlorkohlenstoff gelöst und bei -10°C 8 g (0,113 Mol) Chlor langsam unter Rühren in die Reaktionsmischung eingeleitet. Anschliessend wird eine halbe Stunde bei 0°C und dann eineinhalb Stunden bei 20°C nachgerührt. Dann wird das Lösungsmittel im Vakuum abgezogen und der Rückstand mit 100 ml n-Hexan verrührt. Nach Abkühlen auf 0°C wird der kristalline Feststoff abgesaugt. Man erhält 32,4 g (91% der Theorie) trans-3-(1,2,2-Trichlor-2-(4-chlor-phenyl)-ethyl)-2,2-dimethyl-cyclopropan-1-carbonsäure als farblosen Feststoff mit dem Schmelzpunkt 145–150°C.

$^1$H-NMR-Spektrum in $CDCl_3$/TMS, τ (ppm)

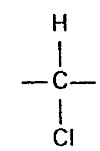 : 5,67–5,95 (m/1 H)

Analog Beispiel 1 erhält man

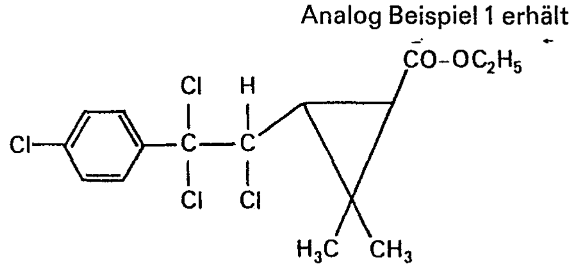

Ausbeute:
90% der Theorie

trans

$^1$H-NMR/$CDCl_3$ τ (ppm)

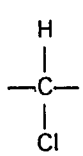 : 5,7–5,95 (m/1 H)

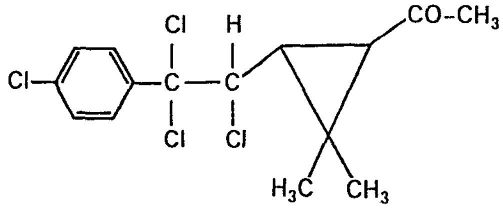

Ausbeute:
85% der Theorie

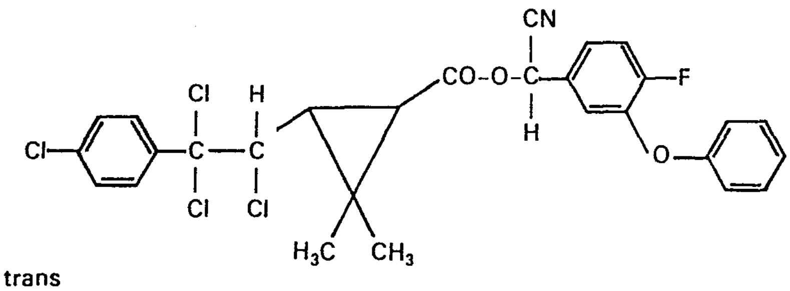

Ausbeute:
88% der Theorie

trans

$^1$H-NMR/$CDCl_3$ τ (ppm)

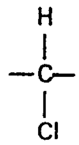 : 5,74-6,05 (m/1 H)

benzyl-H: 3,62-3,85 (m/1 H)

trans

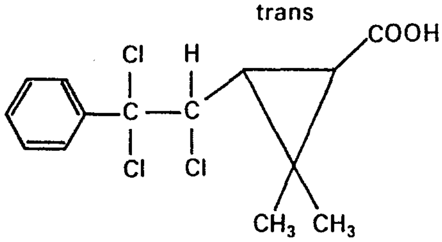

Ausbeute:
88% d. Theorie
Schmp. 148°C

Beispiel 2:

Enthalogenierung:

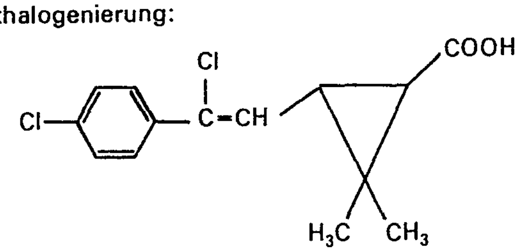

Zu einer Mischung von 100 ml Ethanol und 3,6 g (0,055 Mol) Zinkstaub (= Enthalogenierungsmittel) gibt man unter Rühren bei 50°C portionsweise 17,8 g (0,05 Mol) trans-3-(1,2,2-Trichlor-2-(4-chlor-phenyl)-ethyl)-2,2-dimethyl-cyclopropan-1-carbonsäure (= Halogenverbindung). Anschliessend wird 1 Stunde unter Rühren zum Rückfluss erhitzt. Dann wird auf Raumtemperatur abgekühlt und das Ethanol im Wasserstrahlvakuum abgezogen. Der kristalline Rückstand wird in 150 ml Methylenchlorid aufgenommen und einmal mit 150 ml Wasser, dem 10 ml konz. Salzsäure zugesetzt sind und dann nochmals mit 150 ml Wasser ausgeschüttelt. Die organische Phase wird abgetrennt, über Magnesiumsulfat getrocknet und im Vakuum vom Lösungsmittel befreit. Man erhält 13,7 g (96% der Theorie) trans-3-(Z/E-2-Chlor-2-(4-chlor-phenyl)-vinyl)-2,2-dimethyl-cyclopropan-1-carbonsäure = Endprodukt) als hellgelben Feststoff mit einem Z:E-Verhältnis von 95:5. Das Z/E-Verhältnis wird durch $^1$H-NMR- bestimmt (Verhältnis der Vinyl-Protonen von Z und E).

$^1$H-NMR in $CDCl_3$/TMS τ (ppm):

Z : 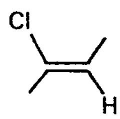 : 4,14 (d)

E . 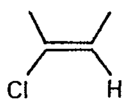 : 4,29 (d)

Analog Beispiel 2 erhält man:

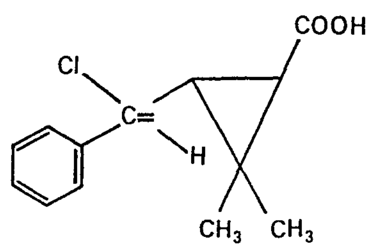

Ausbeute:
95% d. Theorie

trans Z (98%)
E ( 2%)

$^1$H-NMR in $CDCl_3$/TMS, τ (ppm)
Vinyl-H 4,14 (d)/1 H
Dimethyl-H: 8,58 (s)/3 H
8,74 (s)/3 H

Analog den Beispielen 1 und 2 können die in Tabelle I aufgeführten E→ Z Umwandlungen durchgeführt werden.

Tabelle I

(IV) (II) (I)

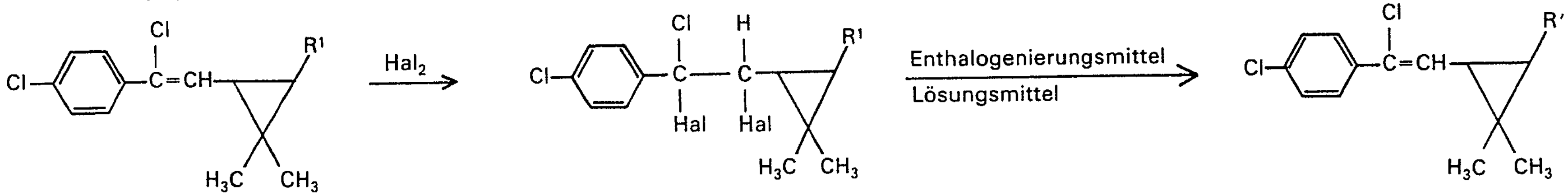


Z/E - 1 RS-trans Ausgangsprodukt — 1 RS-trans Halogenverbindung — E/Z - 1 RS-trans Endprodukt

| Ausgangsprodukt | % Z | % E | Hal | Enthalogenierungsmittel | Lösungsmittel | Temp. Reakt.-Zeit | Endprodukt | % Z | % E | Ausbeute (% der Theorie bezogen auf Halogenverbindungen |
|---|---|---|---|---|---|---|---|---|---|---|
| $R^1 =$ -COOH | 40 | 60 | Cl | Zink-Staub | $C_2H_5OH$ | 80°C 2 Stdn. | R′ = COOH | 95 | 5 | 96 |
| $R^1 =$ -COOH | 0 | 100 | Cl | Zinkstaub | $C_2H_5OH$ | 80°Y 1 Std. | R′ = COOH | 94 | 6 | 83 |
| $R^1 =$ -COOH | 40 | 60 | Cl | Zink-Staub | Dimethylformamid | 80–110°C 2 Stdn. | R′ = COOH | 96 | 4 | 93 |
| $R^1 =$ -COOH | 40 | 60 | Cl | Zink-Staub | $CH_3COOH$ | 100°C 1 Std. | R′ = COOH | 97 | 3 | 89 |
| $R^1 =$ -COOH | 40 | 60 | Br | Zink-Staub | Dimethylformamid | 80–110°C 1 Std. | R′ = COOH | 79 | 21 | 90,6 |
| $R^1 =$ -COOH | 40 | 60 | Br | Zink-Staub | $C_2H_5OH$ | 80°C 1 Std. | R′ = COOH | 77 | 23 | 88 |
| $R^1 =$ -COONa | 40 | 60 | Br | $P[N(CH_3)_2]_3$ | Tetrahydrofuran | 20°C 2 Stdn. 50°C 3 Stdn. | R′ = COOH | 75 | 25 | |
| $R^1 =$ -COOH | 40 | 60 | Br | 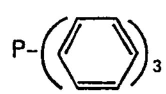 | Toluol | 80°C 4 Stdn. | R′ = COOH | 80 | 20 | 75 |

Tabelle I (Fortsetzung)

| Ausgangsprodukt | %Z | %E | Hal | Enthalogenierungsmittel | Lösungsmittel | Temp. Reakt.-Zeit | Endprodukt | %Z | %E | Ausbeute (% der Theorie bezogen auf Halogenverbindungen |
|---|---|---|---|---|---|---|---|---|---|---|
| $R^1$ = 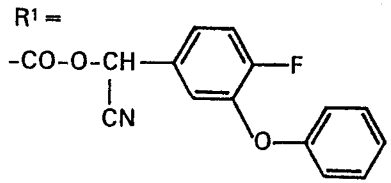 | 40 | 60 | Cl | Zink-Staub | $C_2H_5OH$ | 80°C 1 Std. | R′ = 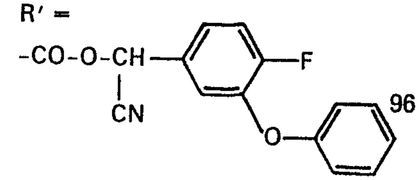 | 96 | 4 | 45 |
| $R^1$ = $-COOC_2H_5$ | 40 | 60 | Cl | Zink-Staub | $C_2H_5OH$ | 80°C 1,5 Stdn. | R′ = $COOC_2H_5$ | 96 | 4 | 55 |
| $R^1$ = $-COOH$ | 40 | 60 | Br | KJ (4 Mol) | $CH_3OH$ | 60°C 4 Stdn. | R′ = COOH | 70 | 30 | 85 |
| $R^1$ = $-COOH$ | 40 | 60 | Br | KJ (4 Mol) | Aceton | 60°C 4 Stdn. | R′ = COOH | 70 | 30 | 80 |
| $R^1$ = 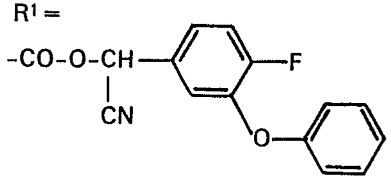 | 40 | 60 | Br | Zink-Staub | $CH_3COOH$ | 80°C 0,5 Stdn. | R′ = 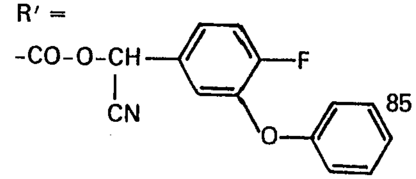 | 85 | 15 | 75 |
| $R^1$ = 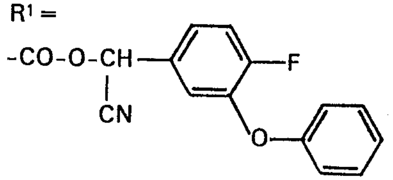 | 40 | 60 | Cl | Zink-Staub | $CH_3COOH$ | 80°C | R′ = 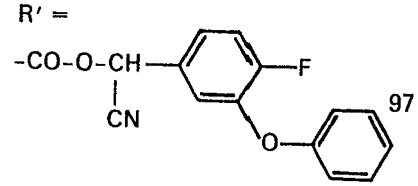 | 97 | 3 | 80 |

Beispiel 3

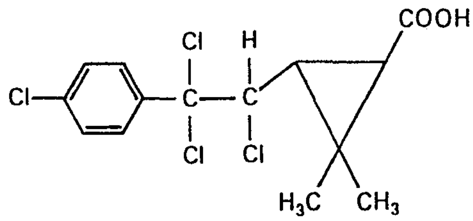

17,7 g (0,05 Mol) trans-3-(E,Z-2-Chlor-2-(4-chlor-phenyl)-vinyl)-2,2-dimethyl-1-acetyl-cyclopropan werden in 50 ml Tetrachlorkohlenstoff gelöst und bei −15°C 8 g Chlor langsam unter Rühren in die Reaktionsmischung eingeleitet. Anschliessend wird eine halbe Stunde bei 0°C und dann 1½ Stunden bei 20°C nachgerührt. Dann wird das Lösungsmittel im Vakum abgezogen. Der zähe ölige Rückstand wird in 25 ml Tetrahydrofuran gelöst und bei 30–35°C zu einer Mischung aus 17,9 g Natriumhydroxid, 90 ml Wasser und 21,4 g Brom, langsam unter Rühren zugetropft. Anschliessend wird 2 Stunden bei Raumtemperatur gerührt. Das Tetrahydrofuran wird dann im Vakuum abdestilliert und der Rückstand mit 20 ml Wasser versetzt. Die wässrige Phase wird 2mal mit 100 ml Methylenchlorid ausgeschüttelt. Dann wird die wässrige Phase mit konz. Salzsäure angesäuert und das ausgefallene Öl 2mal mit 150 ml Methylenchlorid extrahiert. Die vereinigten Methylenchloridphasen werden über Magnesiumsulfat getrocknet und dann das Lösungsmittel im Vakuum abdestilliert. Man erhält 9,6 g (53,9% der Theorie) trans-3-(1,2,2-Trichlor-2-(4-chlor-phenyl)-ethyl)-2,2-dimethyl-cyclopropancarbonsäure als zähes Öl. Die Struktur wird durch das $^1$HNMR-Spektrum bestätigt.

Beispiel 4:

(a)

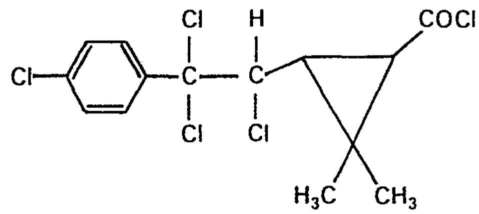

14 g trans-3-(2,2-Dichlor-2-(4-chlor-phenyl)-1-chlor-ethyl)-2,2-dimethyl-cyclopropan-1-carbonsäure werden in 100 ml Tetrachlorkohlenstoff gelöst, mit 75 g Thionylchlorid versetzt und 4 Stunden zum Rückfluss erhitzt. Anschliessend wird das Lösungsmittel und überschüssiges Thionylchlorid im Vakuum abgezogen. Man erhält 13,7 g trans-3-(2,2-Dichlor-2-(4-chlor-phenyl)-1-chlor-ethyl)-2,2-dimethyl-cyclopropan-1-carbonsäurechlorid als zähes Öl.

Analog Beispiel 4 erhält man

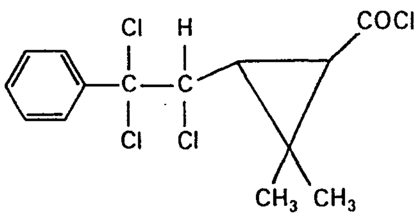

Ausbeute:
93% d. Theorie

(b)

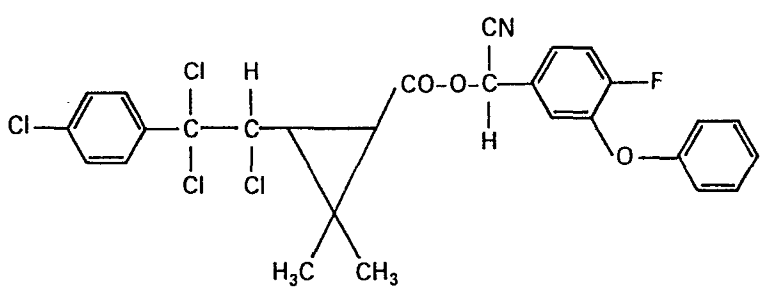

6,36 (0,017 Mol) trans-3-(2,2-Dichlor-2-(4-chlor-phenyl)-1-chlor-ethyl)-2,2-dimethyl-cyclopropan-1-carbonsäure-chlorid und 3,67 g (0,017 Mol) 3-Phenoxy-4-fluor-benzaldehyd werden zusammen unter Rühren bei 20–25°C zu einer Mischung von 1,13 g Natriumcyanid, 1,7 ml Wasser, 100 ml n-Hexan und 0,6 g Tetrabutylammoniumbromid getropft und dann 4 Stunden bei 20–25°C gerührt. Anschliessend wird die Reaktionsmischung mit 300 ml Toluol versetzt und 2mal mit je 300 ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 8,5 g (86% der Theorie) trans-3-(2,2-Dichlor-2-(4-chlor-phenyl)-1-chlor-ethyl)-2,2-dimethyl-cyclopropan-1-carbonsäure-α-cyano-3-phenoxy-4-fluorbenzylester als zähes Öl.

$^1$H-NMR in $CDCl_3$/TMS τ(ppm):

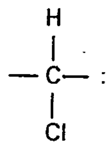 : 5,74-6,05 (m/1 H)

benzyl-H: 3,62–3,85 (m/1 H)

Analog erhält man:

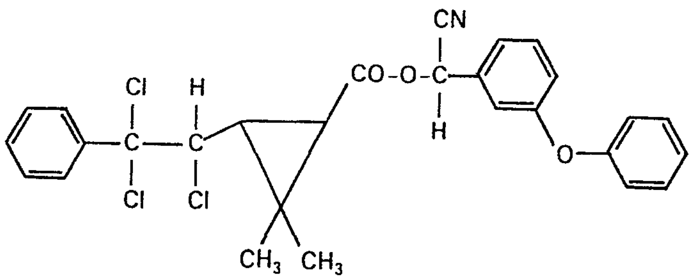

Ausbeute: 87% der Theorie

$^1$H-NMR in $CDCl_3$/TMS, τ(ppm):

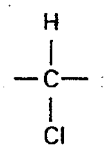 5,72-6,1 (m/1 H)

benzyl-H: 3,64–3,83 (m/1H)

Beispiel A

Test mit Psoroptes cuniculi

Lösungsmittel:

35 Gew.-Teile Äthylenglykolmonoethyläther

35 Gew.-Teile Nonylphenolpolyglykoläther

Zur Herstelung einer zweckmässigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

Etwa 10–25 Psoroptes cuniculi werden in 1 ml der zu testenden Wirkstoffzubereitung gebracht, die in Tablettennester einer Tiefziehverpackung pipettiert wurden. Nach 24 Stunden wird der Abtötungsgrad bestimmt.

Bei diesem Test zeigen die erfindungsgemässen Verbindungen der Herstellungsbeispiele überlegene Wirkung gegenüber dem Stand der Technik.

Beispiel B

Test mit Boophilus microplus resistent

Lösungsmittel:

35 Gew.-Teile Äthylenglykolmonoethyläther

35 Gew.-Teile Nonylphenolpolyglykoläther

Zur Herstellung einer zweckmässigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen die erfindungsgemässen Verbindungen der Herstellungsbeispiele überlegene Wirkung gegenüber dem Stand der Technik.

Beispiel C

Test mit Lucilia cuprina res.-Larven

Emulgator:

35 Gew.-Teile Äthylenglykolmonoethyläther

35 Gew.-Teile Nonylphenolpolyglykoläther

Zur Herstellung einer zweckmässigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen die erfindungsgemässen Verbindungen der Herstellungsbeispiele überlegene Wirkung gegenüber dem Stand der Technik.

**Patentansprüche**

1. Verfahren zur Herstellung von trans-3-(Z-2-Chlor-2-aryl-vinyl)-2,2-dimethyl-cyclopropan-1-carbonsäure-derivaten der Formel (I)

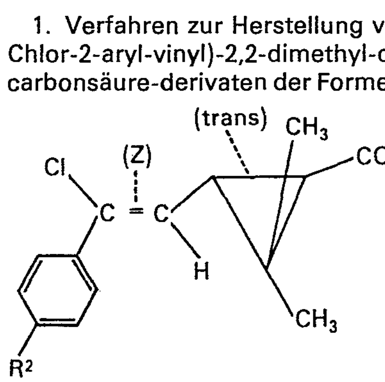

(I)

in welcher
$R^2$ für Wasserstoff oder Chlor und
R für Wasserstoff, ein Alkalimetall oder Erdalkalimetalläquivalent, ein gegebenenfalls substituiertes Ammonium, Alkyl oder gegebenenfalls durch Alkyl, Alkenyl, Alkinyl, Halogen und/oder Cyano substituiertes Phenoxybenzyl steht,
dadurch gekennzeichnet, dass man trans-3-(1,2-Dihalogen-2-chlor-2-aryl-ethyl)-2,2-dimethyl-cyclopropan-1-carbonsäre-derivate der Formel (II)

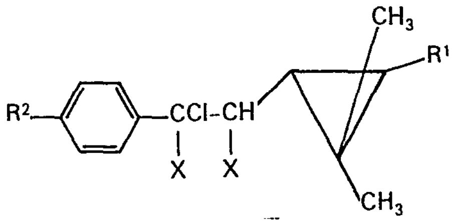
(II)

in welcher
$R^2$ für Wasserstoff oder Chlor steht,
$R^1$ für Cyano, Acetyl, Chlorcarbonyl oder für den Rest -COOR steht, worin R die oben angegebene Bedeutung hat, und
X für Chlor oder Brom steht,
mit einem zur Eliminierung von zwei vicinalen Halogenatomen geeigneten Enthalogenierungsmittel gegebenenfalls in Gegenwart eines Verdünnungsmittels bei Temperaturen zwischen 0 und 150°C umsetzt und gegebenenfalls die Produkte der Formel (I a)

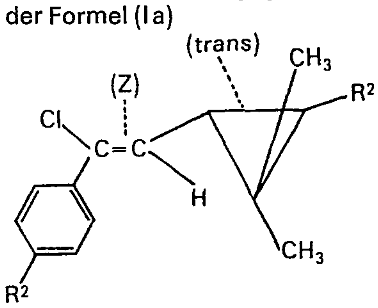
(I a)

in welcher
$R^2$ für Wasserstoff oder Chlor und
$R^3$ für Cyano, Acetyl oder Chlorcarbonyl stehen, nach üblichen Methoden in die Verbindungen der Formel (I) umwandelt oder Verbindungen der Formel (I) nach üblichen Methoden in andere Verbindungen der Formel (I) umwandelt.

2. Trans-3-(1,2,2-Trichlor-2-(4-chlor-phenyl)-ethyl)-2,2-dimethyl-cyclopropan-1-carbonsäure-derivate der Formel (III)

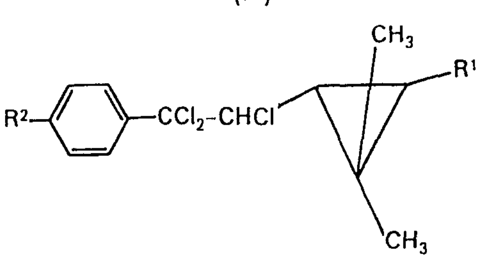
(III)

in welcher
$R^2$ für Wasserstoff oder Chlor steht und
$R^1$ die unter Formel (II) in Anspruch 1 angegebene Bedeutung hat.

3. Verfahren zur Herstellung von Verbindungen der Formel (III)

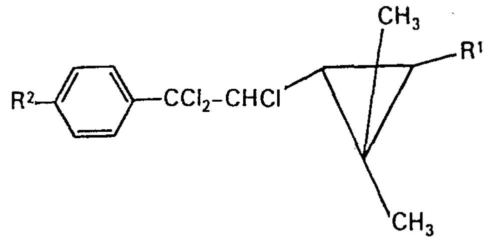
(III)

dadurch gekennzeichnet, das man trans-3-(E/Z-2-Chlor-2-(4-aryl-vinyl)-2,2-dimethyl-cyclopropan-1-carbonsäure-derivate der Formel (IV)

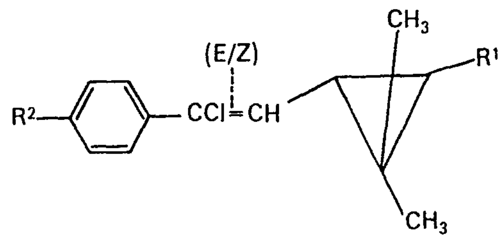
(IV)

in welcher
$R^1$ und $R^2$ die unter Formel (II) in Anspruch 1 angegebene Bedeutung haben,
mit Chlor gegebenenfalls in Gegenwart eines Verdünnungsmittels bei Temperaturen zwischen -50 und +50°C umsetzt und gegebenenfalls anschliessend die Produkte der Formel (III a)

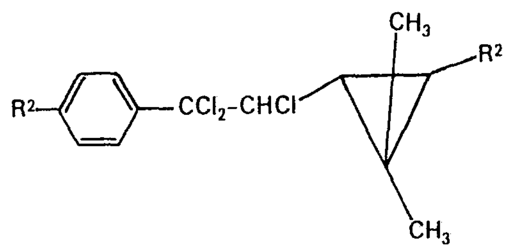
(III a)

in welcher
$R^2$ für Wasserstoff oder Chlor und
$R^3$ für Cyano, Acetyl oder Chlorcarbonyl stehen, nach üblichen Methoden in die Verbindungen der Formel (III),
in welcher
$R^3$ für -COOR steht, umwandelt.

4. Verfahren nach Anspruch 1, dadurch gekennzeichnet, dass man als Enthalogenierungsmittel Metalle verwendet.

5. Verfahren nach Anspruch 1, dadurch gekennzeichnet, dass man als Enthalogenierungsmittel Alkalimetalljodide oder -bromide verwendet.

6. Verfahren nach Anspruch 1, dadurch gekennzeichnet, dass man als Enthalogenierungsmittel Chrom(II)-sulfat oder Kupfer(I)-bromid verwendet.

7. Verfahren nach Anspruch 1, dadurch gekennzeichnet, das man als Enthalogenierungsmittel Triphenylphosphin oder Phosphorigsäuretri-di-methylamid verwendet.

8. Verfahren nach Anspruch 1, dadurch gekennzeichnet, dass man die Enthalogenierung bei Temperaturen zwischen 0°C und 150°C und bei Drucken zwischen 0,1 und 10 bar durchführt.

9. Verfahren nach Anspruch 1, dadurch gekennzeichnet, dass man je Mol Ausgangsverbindung der Formel (II) 1 bis 2 Mol Enthalogenierungsmittel einsetzt.

10. Verwendung von Verbindungen der Formel (III) gemäss Anspruch 2, in welcher
$R^2$ für Wasserstoff oder Chlor und
$R^1$ für gegebenenfalls durch Alkyl, Alkenyl, Alkinyl, Halogen und/oder Cyano substituiertes Phenoxybenzyloxycarbonyl stehen,
als Wirkstoffkomponenten in insektiziden und akariziden Mitteln, insbesondere in Ektoparasitiziden.

**Revendications**

1. Procédé de préparation de dérivés d'acides trans-3-(Z-2-chloro-2-aryl-vinyl)-2,2-diméthyl-cyclopropane-1-carboxyliques de formule (I)

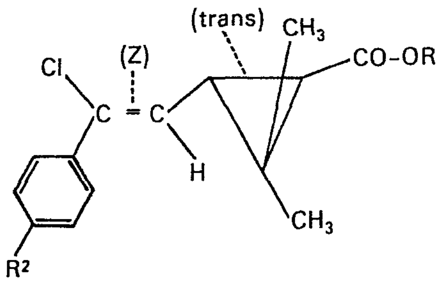

(I)

dans laquelle
$R^2$ représente l'hydrogène ou le chlore et
$R^1$ représente l'hydrogène, un métal alcalin ou un équivalent de métal alcalino-terreux, un groupe ammonium éventuellement substitué, alkyle ou phénoxybenzyle éventuellement substitué par des groupes alkyle, alcényle, alcynyle, des halogènes et/ou des groupes cyano, caractérisé en ce que l'on fait réagir des dérivés d'acides trans-3-(1,2-dihalogéno-2-chloro-2-aryl-éthyl)-2,2-diméthyl-cyclopropane-1-carboxyliques de formule (II)

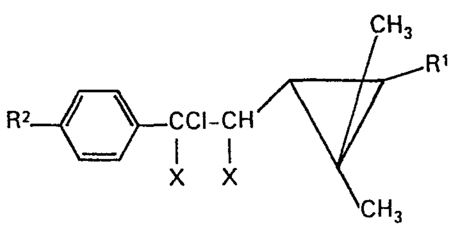

(II)

dans laquelle
$R^2$ représente l'hydrogène ou le chlore,
$R^1$ représente un groupe cyano, acétyle, chlorocarbonyle ou le reste -COOR dans lequel R a la signification indiquée ci-dessus, et
X représente le chlore ou le brome,
avec un agent déshalogénant permettant d'éliminer 2 atomes d'halogène vicinaux, éventuellement en présence d'un diluant, à des températures de 0 à 150°C, et le cas échéant on convertit les produits de formule (Ia)

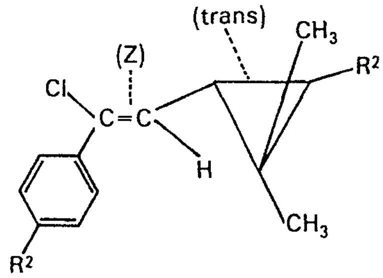

(I a)

dans laquelle
$R^2$ représente l'hydrogène ou le chlore et
$R^3$ représente un groupe cyano, acétyle ou chlorocarbonyle,
par des modes opératoires usuels, en les composés de formule (I), ou bien on convertit les composés de formule (I) par des modes opératoires usuels, en autres composés de formule (I).

2. Dérivés de l'acide trans-3-(1,2,2-trichloro-2-(4-chlorophényl)-éthyl)-2,2-diméthyl-cyclopropane-1-carboxylique de formule (III)

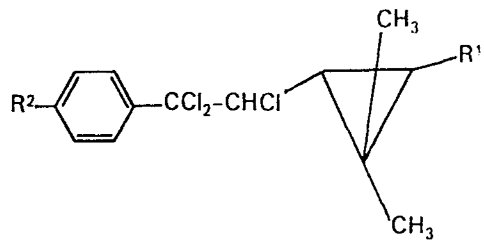

(III)

dans laquelle
$R^2$ représente l'hydrogène ou le chlore et
$R^1$ a la signification indiquée dans la revendication 1 en référence à la formule (II).

3. Procédé de préparation des composés de formule (III)

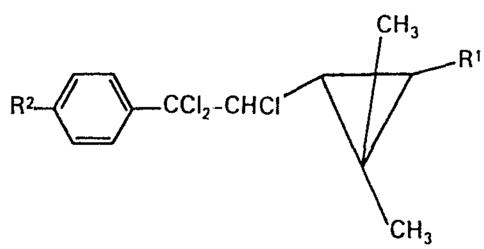

(III)

caractérisé en ce que l'on fait réagir des dérivés d'acides trans-3-(E/Z-2-chloro-2-(4-aryl-vinyl)-2,2-diméthyl-cyclopropane-1-carboxyliques de formule (IV)

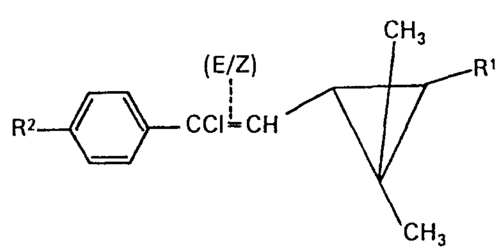

(IV)

dans laquelle
$R^1$ et $R^2$ ont les significations indiquées dans la revendication 1 en référence à la formule (II),
avec le chlore, éventuellement en présence d'un

diluant, à des températures comprises entre -50 et +50°C, et le cas échéant on convertit ensuite les produits de formule (IIIa)

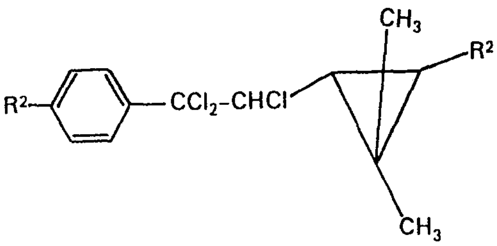

(III a)

dans laquelle
$R^2$ représente l'hydrogène ou le chlore et
$R^3$ représente un groupe cyano, acétyle ou chlorocarbonyle,
par des modes opératoires usuels, en les composés de formule (III)
dans laquelle
$R^3$ représente le reste -COOR.

4. Procédé selon la revendication 1, caractérisé en ce que l'on utilise en tant qu'agents déshalogénants des métaux.

5. Procédé selon la revendication 1, caractérisé en ce que l'on utilise en tant qu'agents déshalogénants des iodures ou bromures de métaux alcalins.

6. Procédé selon la revendication 1, caractérisé en ce que l'on utilise en tant qu'agent déshalogénant le sulfate de chrome-II ou le bromure de cuivre-I.

7. Procédé selon la revendication 1, caractérisé en ce que l'on utilise en tant qu'agent déshalogénant la triphénylphosphine ou le tri-di-méthylamide de l'acide phosphoreux.

8. Procédé selon la revendication 1, caractérisé en ce que l'on effectue la déshalogénation à des températures de 0 à 150°C et sous des pressions de 0,1 à 10 bars.

9. Procédé selon la revendication 1, caractérisé en ce que l'on utilise 1 à 2 moles d'agent déshalogénant par mole du composé de départ de formule (II).

10. Utilisation des composés de formule (III) selon la revendication 2, dans laquelle
$R^2$ représente l'hydrogène ou le chlore et
$R^1$ représente un groupe phénoxybenzyloxycarbonyle éventuellement substitué par des groupes alkyle, alcényle, alcynyle, des atomes d'halogènes et/ou des groupes cyano,
en tant que composants actifs dans des produits insecticides et acaricides, en particulier dans des ectoparasiticides.

## Claims

1. Process for the preparation of trans-3-(Z-2-chloro-2-aryl-vinyl)-2,2-dimethyl-cyclopropane-1-carboxylic acid derivatives of the formula (I)

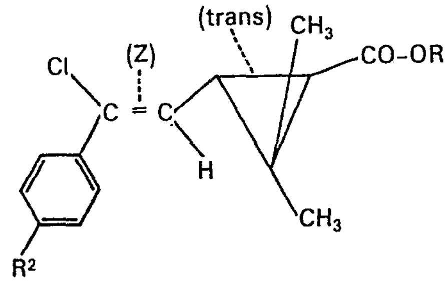

(I)

in which
$R^2$ represents hydrogen or chlorine and
R represents hydrogen, an alkali metal or one equivalent of an alkaline earth metal, an optionally substituted ammonium, alkyl, or phenoxybenzyl which is optionally substituted by alkyl, alkenyl, alkinyl, halogen and/or cyano,
characterised in that trans-3-(1,2-dihalogen-2-chloro-2-aryl-ethyl)-2,2-dimethyl-cyclopropane-1-carboxylic acid derivatives of the formula (II)

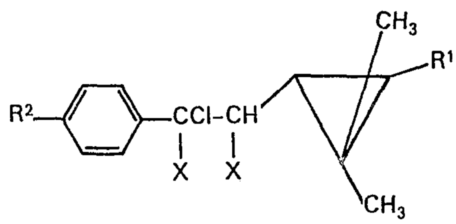

(II)

in which
$R^2$ represents hydrogen or chlorine,
$R^1$ represents cyano, acetyl, chlorocarbonyl or the -COOR radical,
wherein
R has the above mentioned meaning, and
X represents chlorine or bromine,
is reacted with a dehalogenating agent suitable for the elimination of two vicinal halogen atoms, if appropriate in the presence of a diluent, at temperatures between 0 and 150°C, and, if appropriate, the products of the formula (I a)

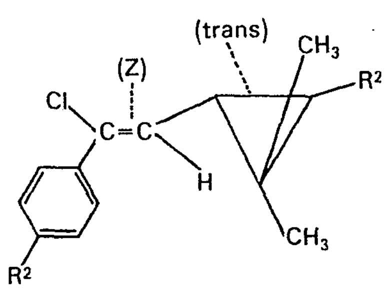

(I a)

in which
$R^2$ represents hydrogen or chlorine and
$R^3$ represents cyano, acetyl or chlorocarbonyl,
are converted into the compounds of the formula (I) by customary methods, or compounds of the formula (I) are converted into other compounds of the formula (I) by customary methods.

2. Trans-3-(1,2,2-Trichloro-2-(4-chloro-phenyl)-ethyl)-2,2-dimethyl-cyclopropane-1-carboxylic acid derivatives of the formula (III)

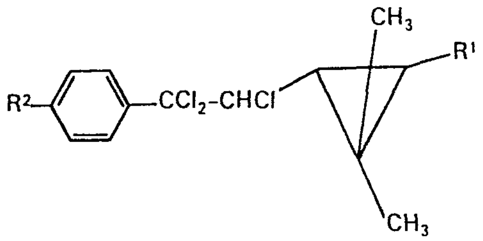

(III)

in which

$R^2$ represents hydrogen or chlorine and

$R^1$ has the meaning given under formula (II) in Claim 1.

3. Process for the preparation of compounds of the formula (III)

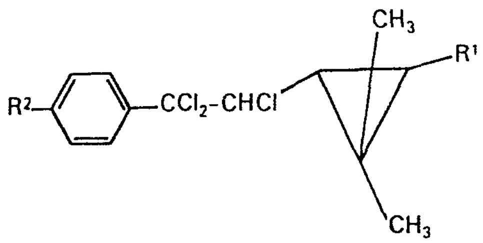

(III)

characterised in that trans-3-(E/Z-2-chloro-2-(4-aryl-vinyl)-2,2-dimethyl-cyclopropan-1-carboxylic acid derivatives of the formule (IV)

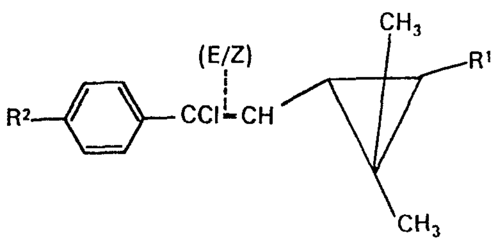

(IV)

in which

$R^1$ and $R^2$ have the meaning given under formula (II) in Claim 1,

are reacted with chlorine, if appropriate in the presence of a diluent, at temperatures between -50 and +50°C and, if appropriate, the products of the formule (III a)

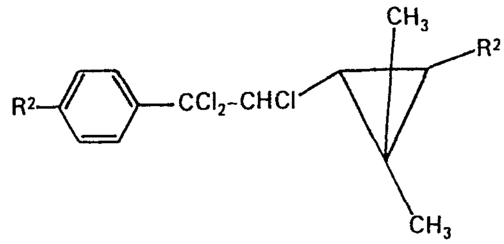

(III a)

in which

$R^2$ represents hydrogen or chlorine and

$R^3$ represents cyano, acetyl or chlorocarbonyl,

are then converted into the compounds of the formula (III) in which $R^3$ represents -COOR, by customary methods.

4. Process according to Claim 1, characterised in that metals are used as dehalogenating agents.

5. Process according to Claim 1, characterised in that alkali metal iodides or bromides are used as dehalogenating agents.

6. Process according to Claim 1, characterised in that chromium-II sulphate or copper-I bromide are used as dehalogenating agents.

7. Process according to Claim 1, characterised in that triphenylphosphine or phosphorous acid tri-di-methylamide are used as dehalogenating agents.

8. Process according to Claim 1, characterised in that the dehalogenation is carried out at temperatures of between 0°C and 150°C and under pressures of between 0.1 and 10 bars.

9. Process according to Claim 1, characterised in that 1 to 2 mols of dehalogenating agent are employed per mol of starting compound of the formula (II).

10. Use of the compounds of the formula (III) according to Claim 2,

in which

$R^2$ represents hydrogen or chlorine and

$R^1$ represents phenoxybenzyloxycarbonyl which is optionally substituted by alkyl, alkenyl, alkinyl, halogen and /or cyano,

as active compound components in insecticidal and acaricidal agents, in particular in ectoparasiticides.